Europäisches Patentamt

European Patent Office

Office européen des brevets

(11) Veröffentlichungsnummer: **0 286 798**

**A2**

(12) **EUROPÄISCHE PATENTANMELDUNG**

(21) Anmeldenummer: 88102246.1

(22) Anmeldetag: 16.02.88

(51) Int. Cl.⁴: **A61M 5/30**

Die Bezeichnung der Erfindung wurde geändert (Richtlinien für die Prüfung im EPA, A-III, 7.3).

(30) Priorität: 17.02.87 HU 61687

(43) Veröffentlichungstag der Anmeldung:
19.10.88 Patentblatt 88/42

(84) Benannte Vertragsstaaten:
AT BE CH DE ES FR GB GR IT LI LU NL SE

(71) Anmelder: **Fegyver- és Gázkeszülékgyár**
**Soroksari ut 158**
**Budapest IX(HU)**

(72) Erfinder: **Erdélyi, Gyula, Dr.**
**Aga ut 6**
**HU-1113 Budapest(HU)**
Erfinder: **Nagy, Lajos**
**Vercse ut 9**
**HU-1213 Budapest(HU)**
Erfinder: **Fejes, Kalmanne**
**Fodor ut 5-7**
**HU-1126 Budapest(HU)**

(74) Vertreter: **Füchsle, Klaus, Dipl.-Ing. et al**
**Hoffmann . Eitle & Partner Patentanwälte**
**Arabellastrasse 4**
**D-8000 München 81(DE)**

(54) **Nadelloses Impfgerät.**

(57) Nadelloses, universales Impfgerät mit veränderlicher Einschußtiefe zur Verwendung beim Impfen in der menschlichen und Kleintierheilkunde, das einen sich am Gerätekörper (32) anschließenden Impfzylinder (18), Impfkolben (19), weiterhin einen Impfstoffendbeschleuniger (8), Impfkopf (9, 10) und Ampullenhalter (3), sowie einen Griff (31) und eine kraftlagernde und rückführende Feder (33) aufweist. Am Gerätekörper (32) ist ein Aufziehgriff (29) angeordnet, der mit der am im Gerätekörper (32) bzw. im Impfzylinder (18) angeordneten Impfkolben (19) anpassenden, rückführenden Feder (33) in Betätigungsverbindung steht. Der Aufziehgriff (39) steht weiterhin mittels des Aufziehschaftes (42) und des kraftlagernden Befestigungsbegrenzers (36) mit der kraftlagernden Feder (40) in Betätigungsverbindung. Diese Feder (40) ist während des Betriebs des kraftlagernden Befestigungsbegrenzers (36), weiterhin des Federgehäuses (34) am verlängerten Schaft des Impfkolbens (19) angeschlossen.

Fig.1

# NADELLOSES, HANDBETÄTIGBARES UNIVERSALES IMPFGERÄT MIT REGELBARER EINSCHUSSTIEFE ZUM EINZELN-UND SERIENIMPFEN VON MENSCHEN UND KLEINTIEREN, BEISPIELSWEISE ZUM EINGE-BEN VON INSULIN

Die Erfindung bezieht sich auf ein nadelloses, handbetätigbares universales Impfgerät mit das Einsaugen des Impfstoffes bremsender Wirkung und Mischmöglichkeit, das mit einer tellerartig ausgebildeten Kraftspeichereinheit betrieben ist und zu präventiven Schutzimpfungen, beziehungsweise zum Eingeben von Insulin in der menschlichen und Kleintierheilkunde gleichmässig geeignet ist.

Mit dem erfindungsgemässen Impfgerät kann die Impfung mittels einmaliger Einstellung der Einschusstiefe - un abhängig von der Impfstoffmenge - mit grosser Genauigkeit serienmässig wiederholt werden, wobei die Veränderung der der Impfstoffmenge entsprechenden Federvorspannung, was bei den bekannten Impfgeräten eine grundsätzliche Vorbedingung ist, weggelassen werden kann.

Es sind derartige Impfgeräte bekannt, deren Betrieb mittels Pressluft, Gaspatrone oder jedwelcher hydraulischen Vorrichtung gesichert ist.

Der gemeinsame Nachteil dieser Geräte zeigt sich in ihrer Kompliziertheit, grossen Abmessungen, in grossem Gewicht und ihrer hohen Herstellungskosten, wodurch ihre Verbreitung erschwert ist.

Das in der DE-PS 1.944.006 beschriebene Impfgerät verfügt über einen Impfzylinder, den den Impfkolben enthält und auch die Ampulle trägt. Sein Betrieb ist mittels einer Patrone gewährleistet.

Durch die ungleichmässige Füllung der Patrone ist aber der ständige Druckwert nicht gesichert. Das Gerät verfügt nicht über Momentauslöseschalter, was bei dem langsamen Ziehen des Abzugshahnes eine nicht entsprechende /langsame/ Ausströmungsgeschwindigkeit des Impfstoffes verursachen kann und das Impfen erfolglos macht.

Die Einschusstiefe kann bei diesem Gerät nicht geregelt werden.

Auf ähnlichem Prinzip arbeitet ein in der DE-PS 3.115.376 beschriebenes Impfgerät mit dem Unterschied, dass das das Impfgerät betätigende Hochdruckgas nicht den Einschuss des Impfstoffes, sondern das Aufziehen einer Feder bewirkt.

Das Aufziehen der Feder erfolgt dem Wert des Gasdruckes entsprechend mit hoher Geschwindigkeit, wodurch nicht möglich ist, den Impfstoff mit anderen Antidoten, eventuell mit Analgetiken zu mischen. Die Einschusstiefe kann nicht einmal bei diesem Gerät geregelt werden.

Die bei dieser Lösung verwendete Spiralfeder übt eine hohe Einschusskraft aus, deshalb kann das Impfgerät nicht universal verwendet werden.

Die US-PS 2.653.602 beschreibt ein Impfgerät, bei dem die Vorspannung der Feder mittels Gas erfolgt, und der Impfstoff nicht eingesaugt, sondern ohne Sicherstellung des fortlaufenden Betriebs eingefüllt wird.

Die nachträgliche Einfüllung des Impfstoffes kann mit Infektionsgefahr und mit ungenauer Dosierung verbunden sein, wobei die Wirksamkeit der Schutzimpfung nicht gesichert ist. Auch bei dieser Lösung kann die Einschusstiefe nicht geregelt werden.

Aus der US-PS 3.526.225 ist ein Impfgerät bekannt, bei dem die Vorspannung der die Impfung durchführenden Feder mittels Pressluft erfolgt.

Die Schwerfälligkeit dieser technischen Lösung ist in erster Reihe durch die komplizierte Zuführung der Pressluft verursacht. Mit dieser Lösung ist die individuelle Selbstimpfung nahezu unmöglich, wodurch die Behandlung der Zuckerkranken mit Insulin nicht gelöst ist, weil nur wenige Haushalte mit Pressluft herstellendem Kompressor versehen sind.

Die Einschusstiefe kann auch bei diesem Gerät nicht geregelt werden, wodurch das Gerät nicht universal verwendet werden kann.

Bei dem Impfgerät nach der US-PS 2.645.223 gelangt der gelagerte Impfstoff mittels Handschaltung eines Zweiwegeventils durch eine Düse in den menschlichen Körper. Das Gerät verfügt nicht über ein Sicherheitsventil, wodurch die Fehlermöglichkeit besteht, dass bei versehentlicher Verstellung des Ventils der Impfstoff nicht durch den Ausschiesskopf in den menschlichen Körper gelangt, sondern in Richtung der Ampulle ausströmt.

Die Einschusstiefe kann nicht einmal bei dieser Lösung geregelt werden.

Die HU-PS 186.718 beschreibt ein mit Siphonpatrone betätigtes Impfgerät, das in erster Reihe zur Impfung von Insulin entwickelt worden ist und zweifellos lückenfüllend im Gesundheitswesen sein könnte.

Nach praktischen Erfahrungen haben aber die Siphonpatrone - wegen ihrer ungleichmässigen Füllung, fehlerhaften Dichtung - den ständigen Druckwert nicht in jedem Fall gewährleistet, oft nicht einmal bei den ersten Einschüssen.

Die Unregelbarkeit der Einschusstiefe, die - schwierige Veränderung der Impfstoffmenge /der Austausch der die Impfstoffmenge regelnden Etalonringe ist nur nach Zerlegen des Impfkopfes möglich/, der nicht stabilisierbare Druckwert verursachte neben Beschädigung des Gewebes auch das Ausfliessen des Impfstoffes, wodurch die Impfung nicht wirksam war.

Das Impfgerät laut der FR-OS 2.549.729 hat ein aus Medikamentraum und Druckraum zusammengesetztes zylindrisches Gehäuse. Die Lösung besteht aus den das Medikament in den Medikamentraum führenden Elementen, aus der das Medikament ausspritzenden Öffnung, aus dem im Medikamentraum schiebbaren Kolben, weiterhin aus Betätigungselementen.

Der grundsätzliche Mangel dieser Lösung besteht darin, dass sie nur zum Einschiessen von Insulin geeignet, also nicht universal ist, die Einschusstiefe kann nicht geregelt werden, so dass die Veränderung der Druckkraft bei verschiedenen Hautparametern nicht möglich ist. Ein weiterer Nachteil besteht darin, dass mehrere Impfstoffe zwecks Einschiessen mit einer einzigen Impfung nicht gemischt werden können.

Das Impfgerät laut EP-PS 01 14 792 ist nur zur subcutan Injektion geeignet, es besteht aus einer Düse, aus einer das flüssige Medikament aufsaugenden und ausspritzenden Einheit, aus einer das Saugen und Ausspritzen regelnden Einheit, aus die Medikamentdose einstellenden Elementen und dem die Ampullen tragenden Bauteil.

Der grundsätzliche Mangel dieser Lösung besteht darin, dass zwar die Menge des in den Impfraum aufsaugbaren Impfstoffes einstellbar ist, die Möglichkeit der fortlaufenden Einstellung beim nacheinanderfolgenden Aufsaugen von mehreren Impfstoffen /Mischmöglichkeit/ nicht gewährleistet ist, damit der Impfstoff im Impfraum gemischt wird.

Ein weiterer Nachteil der Lösung mit Federkraftspeicherung besteht darin, dass das Impfgerät nur subcutan Injektion geeignet /also nicht universal/ ist, so dass die verwendete Federkraftspeicherung zur Ausübung grösserer Kräfte zwecks stärkeren Einschusses /intramuscular/ nicht entsprechend ist.

Das bekannte Impfgerät enthält auch derartige Elemente /Spiralfeder/, deren Federcharakteristik nicht sichern kann, dass sich die Federkraft während der Hublänge zwischen der aufgezogenen und Ausschusslage nur in minimalem Mass verändert.

Nach der Lösung sollte das Problem mittels Erhöhung der Aufziehkraft gelöst werden /zwecks Aufhebung des Medikamentverfliessens/, nach praktischen Erfahrungen verursacht das aber bedeutende Beschädigung des Gewebes, nahe unstillbare Sugillation.

Die bei dieser Lösung nötige hohe Aufziehkraft ist auch durch den Antriebsarm symbolisiert, der am Impfgerät ausdrahbar /kurbelartig/ ausgebildet ist.

Bei diesem Impfgerät besteht weiterhin der Nachteil, dass die Spiralfeder bei der Ermüdung der betätigenden Federkraft relativ oft ausgetauscht werden soll.

Für das Impfgerät laut der Patentschrift Nr. 21 73 106 ist charakteristisch, dass es einen mit Ausspritzdüse versehenen Kopf, einen Flüssigkeitslager, ein den Lager mit der Düse verbindendes Kanalsystem, ein die Düse abschliessendes Elementsystem, weiterhin einen am Gerätekörper gelenkartig befestigten, einen Druck ausübenden Hebel hat. Bei dieser Lösung kann der Druckwert, weiterhin die Menge des flüssigen Stoffes geregelt werden.

Bei der Entwicklung dieses Gerätes hat man danach bestrebt, dass Impfungen von grosser Zahl - auf eine Zeitperiode bezogen - in Serie möglich sei. Das wurde in erster Reihe mittels den grossen Abmessungen der Mechanismus des Impfgerätes, weiterhin mit dem am Tisch montierbaren, eine Aufziehkraft erzeugenden Hebel erreicht.

Das bekannte Impfgerät verfügt über den Nachteil, dass es die kalibrierbare Mischung des Impfstoffes bei dem Aufsaugen in den Impfraum nicht gewährleistet, weil das Aufsaugen nur in einem Takt /nicht gebremst/ der eingestellten Menge entsprechend erfolgt.

Ein anderer Nachteil der bekannten Lösung besteht darin, dass das Impfgerät nicht durch nur eine Person behandelt werden kann, weil zur Betätigung mindestens zwei Hände einer zweiten Person nötig sind.

Im Falle von Montierung, bzw. Befestigung des Impfgerätes am Tisch ist aber die sogenannte Selbstimpfung unmöglich, was eine Bedingung der Insulineingabe ist.

Die erwähnte Patentschrift beschreibt zwar die Betätigung des in der hand gehaltenen Impfgerätes, das ist aber wegen des grossen Gewichtes zweifelhaft, was bei Bewegung während der Impfung Hautbeschädigung verursachen kann.

Die EP-PS 01 19 286 beschreibt ein mit Druckmittel betätigtes Impfgerät, das einen im zylindrischen Gehäuse bewegenden, mit dem Saugkolben zusammenarbeitenden Arbeitskolben, einen am Gerätekörper gelenkartig befestigten handbetätigten Arm und eine die Bewegung des Arbeitskolbens vom Druckwert abhängig lösende Arretierungskonstruktion aufweist.

Der grundsätzliche Nachteil dieser Lösung besteht darin, dass das Aufsaugen von mehreren Impfstoffen in den Impfraum /kalibrierbar und gemischt/ nicht gesichert ist, weil das Aufsaugen nur in einem Takt der eingestellten Menge entsprechend erfolgt.

Ein weiterer Nachteil besteht darin, dass zur Betätigung des Impfgerätes äussere Energie nötig ist, ohne die das Impfgerät nicht betriebsfähig ist. Durch diese Kennzeichen ist das Impfgerät ortsgebunden.

Durch die Kosten der Betätigungsenergie sind die Selbstkosten des Impfgerätes bedeutend erhöht, was die individuelle Verwendung zweifelhaft macht. Die Verwendung dieser Lösung ist in erster Reihe in Krankenhäusern, Kliniken begründet.

Bei den oben bekanntgemachten Impfgeräten ist also die zum Ausschiessen des Impfstoffes nötige Energie in breiten Varianten erzeugt.

Die Vorbedingungen der Betätigung sind in allen Fällen mittels Zwischenschaltung eines Hilfsmittels für die die Arbeit durchführende Spiralfeder gesichert, wobei das Hilfsmittel eine Ölpumpe, Pressluft oder eine Gaspatrone sein kann. Je mehr verbreitet werden die Lösungen grossen Gewichtes und Volumens /z.B. Patentschrift Nr. 21 73 106/, weiterhin die Federvorspannung mittels Antriebsarmen, die die Regelbarkeit der Einschusstiefe zweifelhaft machen.

Durch die Unregelbarkeit der Einschusstiefe, den Mangel an Möglichkeit der Impfstoffmischung, die Nichteinstellbarkeit der Impfstoffmenge während der Impfung, die nicht vollwertige Regelbarkeit der gleichen Einschusskraft wird das Misstrauen an diesen Impfgeräten weiter erhöht.

Die bei den oben bekanntgemachten Impfgeräten festgestellten technischen Mangelhaftigkeiten wurden letzten Endes Hindernisse der breiteren Verwendung solcher Impfgeräte.

Aufgrund der Erkennung dieser Tatsachen ist Zielsetzung unserer Erfindung, die Nachteile der bekannten Impfgeräte zu beseitigen, das Impfgerät nach der HU-PS 186.718 weiterzuentwickeln, und eine Lösung zu schaffen, die geeignet ist:
- zu universalen Einzeln-und Serienimpfung von Menschen und Kleintieren, z.B. zum Eingeben von Insulin ohne Verwendung verschiedener Hilfsmittel /Ölpumpe, Antriebsarm, Siphonpatrone, Pressluft/, mit der Hand in betriebsfertigen Zustand bringend, neben gleichzeitiger und ge bremster Sicherung des Aufsaugens und der Aufsaugvorbereitung;
- zur entsprechenden Regelung der Einschusstiefe bei Einzeln-und Serienimpfungen den Bedürfnissen entsprechend, z.B. intracutan, subcutan, intramuscular;
- zur einmaligen Einstellung des Impfstoffes oder Einstellung des Impfstoffes während Betrieb, eventuell zur Mischung des Impfstoffes ohne Zerlegen des Impfgerätes;
- zur Sicherung der gleichen Einschusskraft unabhängig von der aufzusaugenden Impfstoffmenge, ohne wiederholte Einstellung der Federkraft.

Anhand des universalen Charakters unserer Erfindung ist durch Erreichen der oben aufgezählten Zielsetzungen die Verwendung von präventiven Schutzimpfungen zur Bekämpfung von epidemischen Krankheiten /Typhus, Paratyphus, Tetanus und Toxins kombiniert, Dyphterie, Kinderparalyse, Cholera, Gelbfieber, Influenza, Tollwut usw./, zur Behandlung von chemisch Beschädigten mit Antidoten, zum Eingeben von Analgetiken als Schock-Prävention bei Brand-und anderen Beschädigungen, weiterhin zum Eingeben von Insulin bei Zuckerkranken ermöglicht.

Unserer Erfindung liegt die Erkenntnis zugrunde, dass mit Hilfe des universalen Impfgerätes über die individuelle Zuckerkrankenbehandlung hinaus eine breite Verwendung /Menschen und Kleintiere/ im Gesundheitswesen in der Heilkunde ermöglicht wird, und zwar neben bedeutender Ersparung an Stoff /Impfnadel/ und Arbeitskraft /Krankenhäuser, Kliniken/ bis zur Erhöhung des Wirkungsgrades.

Bei der Impfung von Zuckerkranken wurde die Menge des Insulins erstaunlicherweise nach einer Periode von 3-4 Wochen der Impfung mit nadellosem Impfgerät verminderbar, was in erster Reihe die Folge der hervorragenden Zerstäubung des eingeschossenen Impfstoffes /Impfkopfbehandlung mit Laser/ und der danach folgenden wirksameren Absorbierung war.

Das erfindungsgemässe Impfgerät besteht aus einem am Gerätekörper angeschlossenen Impfzylinder, Impfkolben, mit Impfstoffendbeschleuniger ergänztem Impfkopf, Ampullenhalter, dem die Handhabung des Impfgerätes ermöglichenden und die Abzugeinheit enthaltenden Griff, der kraftlagernden und rückführenden Feder.

Das erfindungsgemässe Impfgerät kann dadurch gekennzeichnet werden, dass am Gerätekörper ein Aufziehgriff von vom Aufziehschaft grösseren Durchmesser angeordnet ist. Mit dieser Lösung kann die Grösse des Aufziehmomentes - ohne Verwendung von Hilfskraft - in vorteilhaftem Mass verändert werden.

Der Aufziehgriff ist mit der rückführenden Feder, die am im Gerätekörper bzw. im Impfzylinder angeordneten Impfkolben angepasst ist, in Betätigungsverbindung, derweise ist das gebremste Aufsaugen des Impfstoffes ermöglicht.

Der erwähnte Aufziehgriff ist gegebenenfalls mit der kraftlagernden Feder ebenfalls in Betätigungsverbindung, die am verlängerten Schat des Impfkolbens angepasst ist.

Am Gerätekörper ist weiterhin eine die Mischung einstellende Kalibrierungskonstruktion angeordnet, deren Befestigungsschraube am Befestigungsbolzen des Mischungeinstellers angepasst ist.

Dadurch, dass das Federgehäse und der kraftlagernde Befestigungsbegrenzer aus gesonderten Körpern hergestellt und während Funktionierung voneinander unabhängig sind, dringt der aufzusaugende Impfstoff von der Menge unabhängig mit gleicher Einschusskraft in den menschlichen Körper.

Die erfindungsgemässe technische Lösung wird nachstehend anhand einer zweckmässige Ausführungsform mit Hilfe der beiliegenden Zeichnungen näher erläutert. Es zeigt

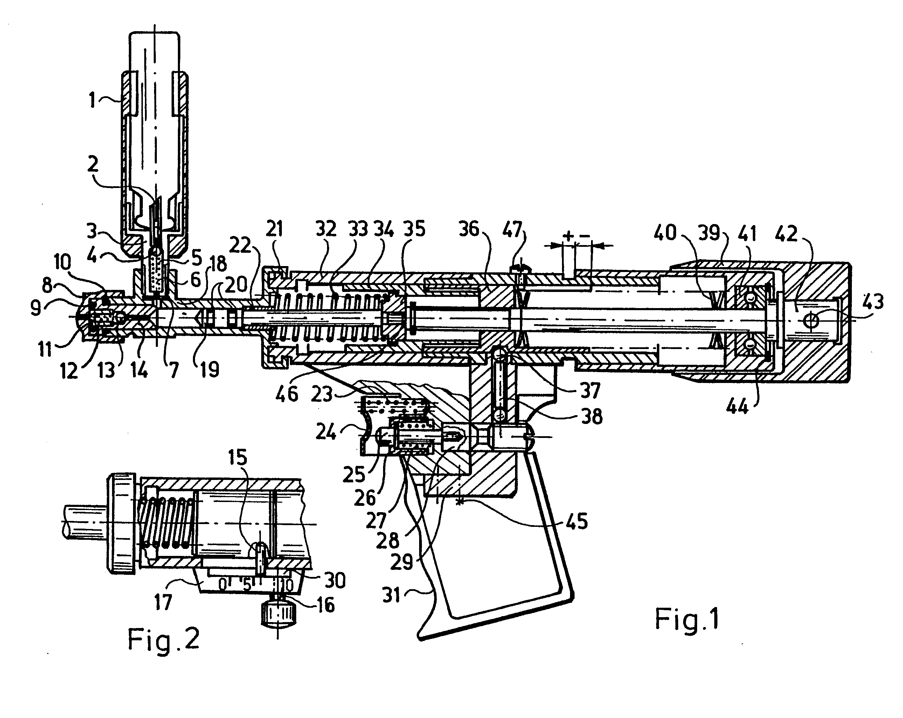

Fig. 1 - den Längsschnitt des erfindungsgemässen Impfgerätes,

Fig. 2 - die am erfindungsgemässen Impfgerät montierte Impfstoffeinstelleinheit.

An einem Gerätekörper 32 ist von vorne ein Impfzylinder 18, von hinten ein Aufziehgriff 39, von unten ein Griff 31 angeschlossen.

Am Aufziehgriff 39 ist ein Einschusstiefenregler 44 angeschlossen. Der Augziehgriff 39 schliesst sich mittels einer an einem Aufziehschaft 42 mit entsprechender Steigung ausgebildeten flachen Gewinde an einem kraftlagernden Befestigungsbegrenzer 36 an, wobei in einer an dessen oberen Teil ausgebildeten längsseitigen Nut eine im Gerätekörper 32 eingeschraubte, in einem Bolzen 47 endende Schraube angeordnet ist, wodurch gesichert ist, dass der kraftlagernde Befestigungsbegrenzer 36 sich in Achsenrichtung bewegen, aber nicht verdrehen kann.

Im Griff 31 ist ein Anlassbolzenhalter 29 angeordnet.

In einem Federgehäuse 34 des Gerätekörpers ist eine Mutter 35 mit Flansch angeordnet, die sich an einer rückführenden Feder 33 anstützt.

Im Impfzylinder 18 ist ein Impfkolben 19 angeordnet, der zwecks besserer Dichtung einen Teflonring 20 enthält, bzw. zwecks Empfang der Dichtungsringe mit einer oder mehreren Nuten ausgebildet ist.

Der Impfkolben 19, der den dichtenden Teflonring 20 trägt, ragt im Innenraum der rückführenden Feder 33 in das Federgehäuse 34 des Gerätekörpers 32 hinein und ist an einem Befestigungsring 46 angeschlossen.

An anderen Ende des Impfzylinders 18 ist ein Impfkopf-Impfstoffendbeschleuniger mittels eines Impfstoffvorbeschleunigers 14, eines Rückschlagventilfederhalters 11, einer Rückschlagventilfeder 12, einer Rückschlagventilkugel 13 angeschlossen.

Der Aufziehgriff 39 des Gerätekörpers 32 ist mit Hilfe eines Befestigungsbolzens am Aufziehschaft angeschlossen, der mit dem kraftlagernden Befestigungsbegrenzer 36 befestigt ist. Am Aufziehschaft 42 ist ein Fusslager 41 angeschlossen, der die leichtere Behandlung des Aufzieh griffs 39 ermöglicht. Der Befestigungsring 46 ist am Aufziehschaft 42 angeordnet.

Der kraftlagernde Befestigungsbegrenzer 36 steht mittels des Federgehäuses 34 mit der Mutter 35 mit Flansch in Verbindung, die durch die rückführende Feder 33 mit dem Impfkolben 19 gleichzeitig bewegt ist.

Im oben beschriebenen Takt bewegen sich die obigen Elemente gemeinsam nach hinten, bis sich der Mischungeinstellbefestigungsbolzen 15 im Federgehäuse 34 am Einstellanstossblock 30 stosst. Danach bewegt sich nur der kraftlagernde Befestigungsbegrenzer 36 nach hinten, sich vom Federgehäuse 36 trennend, bis die daran ausgebildete keilartige Nut in die Linie der oberen Befestigungsbegrenzerkugel 37 gelangt.

Der Einschusstiefenregler regelt die Vorspannung der kraftlagernden Feder 40.

Der Einschusstiefenregler 44 ist am Gerätekörper 32 mit einer scharfen Gewinde angeschlossen, wodurch der Federraum geändert und vom Aufziehgriff 39 unabhängig einstellbar ist.

Die Durchführung des Einschiessens ist durch das Einspringen der Befestigungsbegrenzerkugel 37 in die Nut des kraftlagernden Befestigungsbegrenzers 36 gesichert.

Im Griff 31 ist der die Aufzieh-oder Anlasskonstruktion enthaltende Anlassbolzenhalter 29 befestigt, der durch Anlassbolzenhalterschrauben 45 befestigt ist.

Im Anlassbolzenhalter 29 ist ein Abzug 24 bewegbar angeordnet, der durch eine Abzugstützfeder 23 gesichert ist.

Hinter dem Abzug 24 ist ein Anlassknopf 25 angeordnet, in dem von innen eine in einem Befestigungsfederhalter 26 angeordnete Befestigungsfeder 27 mit einem Ende angestützt ist. Durch den Anlassknopf '25 ist ein befestigungsbegrenzender Zwischenlagebolzen 38 mit Hilfe der Befestigungsbegrenzerkugel 37 gesteuert.

Die Befestigungsfeder 27 ist mit ihrem anderen Ende an einem im Ende eines Anlassbolzens ausgebildeten Federnest angepasst.

Am Gerätekörper 32 sind der Mischungeinstellbefestigungsbolzen 15, eine Mischungeinstellbefestigungsschraube 16, weiterhin ein Mischungeinstellkalibrierer 17 befestigt, mit derer Hilfe die Mischung der Impfstoffe ermöglicht ist, weil sich die äussere zylindrische Fläche des kraftlagernden Befestigungsbegrenzers 36 ohne Hindernis am Befestigungsbegrenzerkugel 37 bewegt.

Eine Saugventilkugel bewegt sich beim Aufsaugen gegen eine in einem Saugventilfedergehäuse 6 angeordnete Saugventilfeder 5 nach unten, und die Rückschlagventilkugel 13 bewegt sich beim Ausschiessen gegen die Rückschlagventilfeder nach vorne.

Die Funktionierung des universalen Impfgerätes beim Aufsaugen des Impfstoffes ist wie folgt:

Wir bringen das Impfgerät zweckmässig am Griff haltend mit der Drehung des Aufziehgriffes 39 im Uhrzeigersinn in den Zustand, indem es den Impfstoff aus der Ampulle aufsaugt und wir bringen gleichzeitig das Impfgerät in den zum Einschiessen aufgezogenem Zustand.

## Aufsaugfunktion:

Beim Verdrehen des Aufziehgriffs im Uhrzeigersinn bewegt sich der kraftlagernde Befestigungsbegrenzer 36 nach hinten und demzufolge wird die Mutter 35 mit Flansch, die durch die rückführende Feder 33 in Richtung des Saugtaktes nach hinten bewegt ist, den Impfkolben 19 nach hinten bewegen.

Infolge des derart entstandenen Vakuums wird die vorangehend eingestellte Impfstoffmenge bei der Rückbewegung der Saugventilkugel 4 gegen die Feder aus der Ampulle durch eine Saugventildichtung 7 in den Impfzylinder 18 eingelassen.

Wenn der kraftlagernde Befestigungsbegrenzer 36 während der Bewegung nach hinten in derjenige Lage gelangt, dass die Befestigungsbegrenzerkugel 37 in die daran ausgebildete Nut einspringt, ist das Aufsaugen des Impfstoffes beendet.

Wenn wir Impfstoffmischung verwenden, ist das durch den kraftlagernden Befestigungsbegrenzer 36 nicht beeinflusst, denn anhand seiner Dimensionierung gelangt dieser nur am Ende des Aufsaugtaktes in einen befestigten Zustand.

Der Mischungeinstellkalibrierer 17, der beliebig an der rechten oder linken Seite des Gerätekörpers 32 angeordnet werden kann, dient bei geringem Bedürfnis an Impfstoff, oder der Einstellung des Impfstoffes der erwünschten aufzusaugenden Menge entsprechend.

Der Mischungeinstellkalibrierer 17 löst die Mischbarkeit des Impfstoffes dadurch, dass im Aufsaugtakt auch der Mischungeinstellbefestigungsbolzen 15 mit dem Federgehäuse 34 sich nach hinten bewegt, bis dieser sich der Kalibrierung entsprechend vorangehend am mittels der Mischungeinstellbefestigungsschraube 16 befestigten Einstellanstossblock 30 anstosst.

Wenn wir zur gleichen Impfung neueren Impfstoff beimischen wollen, wird durch die Lösung gesichert, dass wir - das Drehen des Aufziehgriffs 39 nach rechts anhaltend - die Ampulle im Ampullenhalter 3 austauschen, zugleich die Mischungeinstellbefestigungsschraube 16 auflockern, dann den Einstellanstossblock 30 in einer neueren Lage einstellend befestigen, und das Aufsaugen des Impfstoffes fortsetzen, jetzt aus der neuen einzumischenden Ampulle.

Durch die Lösung ist gesichert, dass vor dem Mischen das Federgehäuse 34, bzw. der sich damit gemeinsam bewegende Mischungeinstellbefestigungsbolzen 15 in unveränderter Lage bleiben, sogar wenn wir den Einstellanstossblock 30 in eine andere Kalibrierungslage bringen.

Aus den vorangehenden folgt, dass unabhängig davon, auf welche Impfstoffmenge der Einstellanstossblock 30 eingestellt wurde oder eventuell mehrere Impfstoffe während des Aufsaugens gemischt wurden, der kraftlagernde Befestigungsbegrenzer 36 bei jedem Aufsaugen mittels Verdrehen des Aufziehgriffes 39 nach rechts bis zur Linie der Befestigungsbegrenzerkugel 37 nach hinten gezogen werden soll.

Das ist nötig, weil das Anlassen des Einschusses dadurch ermöglicht ist, dass der kraftlagernde Befestigungsbegrenzer 36 in eine befestigte Lage gebracht ist.

## Einschussvorbereitungsfunktion:

Damit wir den Impfstoff mit entsprechender Geschwindigkeit und Druck unter die Hautfläche bringen können, soll eine Anfangsgeschwindigkeit gesichert werden, die der gegebenen Impffunktion entspricht.

Das erfindungsgemässe universale Impfgerät kann das mit Hilfe der kraftlagernden Einheit mit Feder erreichen.

Damit wir das Impfgerät in Einschusszustand bringen können, soll das Impfgerät mittels Verdrehen des Aufziehgriffs 39 im Uhrzeigersinn bis zum Anstossen in aufgezogenen Zustand gebracht werden.

Wir haben diese Operation bereits während der Aufsaugfunktion durchgeführt, die beiden Funktionen sind also zweckmässig miteinander kombiniert.

Ausserordentlich wichtig ist bei dieser Operation die Rolle der vorderen Richtung 9 und der hinteren Dichtung 10 des Impfkopfes, die im wesentlichen die Einströmung der sogenannten "falschen" Luft in den Impfzylinder 18 verhindern, was die Operation, das Ergebnis der Impfung nachteilig beeinflussen würde.

Mit Rücksicht darauf, dass das erfindungsgemässe Impfgerät für verschiedene Impffunktionen gebraucht werden kann, weiterhin wegen der unterschiedlichen Hautdicken kann es nötig sein, dass das Impfgerät auch eine im Vergleich zum maximal aufgezogenen Zustand geringere Einschusskraft sichern kann.

Das kann dadurch erreicht werden, dass wir die Vorspannung der kraftlagernden Feder 40 mittels Verdrehen des Einschusstiefenreglers 44 nach rechts oder nach links verdrehend verändern.

Der Kontrolle des Masses der Vorspannung dient die am Einschusstiefenregler 44 ausgebildete Kalibrationseinteilung, die in beiden Richtungen zeigt, welche Vorspannung verwendet wird.

Die Vorspannung kann in jedwelchem Zustand des Impfgerätes, also auch im aufgezogenen Zustand oder im Zustand nach dem Einschiessen eingestellt werden.

Damit das Einschiessen durchgeführt werden kann, soll die Bewegung des Aufziehgriffs 39 und des damit in Verbindung stehenden Aufziehschaftes 42 nach vorne gesichert werden. Dass kann dadurch erreicht werden, dass wir nach dem Aufziehen, also nach dem Einspringen der Befestigungsbegrenzerkugel 37 in die Nut des kraftlagerndem Befestigungsbegrenzers 36 den Aufziehgriff 39 gegen den Uhrzeigersinn bis zur Länge des Betätigungshubs, also um 17,5 mm zurückdrehen, wobei diese Länge am Einschusstiefenregler 44 markiert ist.

Die Funktionierung des universalen Impfgerätes beim Einschiessen des Impfstoffes:

Wir setzen das mit dem Impfstoff aufgefüllte und sich im aufgezogenen Zustand befindende Impfgerät mit dem Ziehen des Abzugs 24 in Betrieb. Beim Ziehen bewegt sich der Anlassbolzen 28 mit dem Abzug 24 gemeinsam bis zur Mittellinie des befestigungsbegrenzenden Zwischenlagebolzens 38 gegen die Abzugstützfeder 23.

Hierbei sinkt die untere Befestigungsbegrenzerkugel 37 bis zum halben Durchmesser in die am Anlassbolzen 28 ausgebildete umlaufende Nut. Dadurch bewegt sich die obere Befestigungsbegrenzerkugel 37 ebenfalls um einen halben Durchmesser nach unten. /Die untere und obere Kugel sind mit demselben Zeichen bezeichnet./

Mit dieser Bewegung ist die Sperrung des kraftlagernden Befestigungsbegrenzers 36 aufgehoben, also die kraftlagernden Feder 40 stossen den kraftlagernden Befestigungsbegrenzer 36 mit grosser Geschwindigkeit nach vorne, welcher die Mutter 35 mit Flansch und damit den Impfkolben 19 vor sich schiebt.

Der Impfkolben 19 stosst bis zum konischen Sitz des Impfzylinders 18 auf und der Impfstoff strömt dabei durch den Impfstoffvorbeschleuniger 14 und den Impfkopf-Impfstoffendbeschleuniger 8 mit grosser Geschwindigkeit aus.

Nach praktischen Erfahrungen ist es nötig, die Sterilisierung des Impfgerätes bei fortlaufendem Gebrauch jede zweite Woche durchzuführen, und zwar derart, dass wir den Impfzylinder 18 mit Hilfe eines schnellschliessenden Bajonettverschlusses 21 mit Verdrehen gegen den Uhrzeigersinn abheben, dann die Sterilisierung durchführen.

Nach der Sterilisierung drehen wir den Impfzylinder 18 mit Hilfe des schnellschliessenden Bajonettverschlusses im Uhrzeigersinn, bis der Gerätekörper 32 am Anstossbegrenzer 22 angepasst ist.

Die Erfindung ist nicht auf die beispielsweise Ausführungsform begrenzt.

Liste der Bezugzeichen

1 - Ampullenhülse
2 - Ampullenstecknadel
3 - Ampullenhalter
4 - Saugventilkugel
5 - Saugventilfeder
6 - Saugventilfedergehäuse
7 - Saugventildichtung
8 - Impfkopf-Impfstoffendbeschleuniger
9 - Impfkopf, vordere Dichtung
10 - Impfkopf, hintere Dichtung
11 - Rückschlagventilfederhalter
12 - Rückschlagventilfeder
13 - Rückschlagventilkugel
14 - Impfstoffvorbeschleuniger
15 - Mischungeinstellbefestigungsbolzen
16 - Mischungeinstellbefestigungsschraube
17 - Mischungeinstellkalibrierer
18 - Impfzylinder
19 - Impfkolben
20 - Impfkolben, Teflondichtung
21 - schnellschliessender Bajonettverschluss
22 - Anstossbegrenzer
23 - Abzugstützfeder
24 - Abzug
25 - Anlassknopf
26 - Befestigungsfederhalter
27 - Befestigungsfeder
28 - Anlassbolzen
29 - Anlassbolzenhalter
30 - Einstellanlassblock
31 - Griff
32 - Gerätekörper
33 - rückführende Feder
34 - Federgehäuse
35 - Mutter mit Flansch
36 - kraftlagernder Befestigungsbegrenzer
37 - Befestigungsbegrenzerkugel
38 - befestigungsbegrenzender Zwischenlagebolzen
39 - Aufziehgriff
40 - kraftlagernde Feder
41 - Fusslager
42 - Aufziehschaft
43 - Befestigungsbolzen
44 - Einschusstiefenregler
45 - Anlassbolzenhalterschraube
46 - Befestigungsring
47 - in Bolzen endende Schraube

**Ansprüche**

1. Nadelloses universales Impfgerät mit veränderlicher Einschusstiefe zur Verwendung beim Impfen in der menschlichen und Kleintierheilkunde, des einen sich am Gerätekörper anschliessenden Impfzylinder, Impfkolben, weiterhin einen Impfstoffendbeschleuniger, Impfkopf und Ampullenhalter, sowie einen Griff und eine kraftlagernde und rückführende Feder aufweist, **dadadurch gekennzeichnet,** dass am Gerätekörper /32/ ein Aufziehgriff /39/ angeordnet ist, der mit der am, im Gerätekörper /32/ bzw. im Impfzylinder /18/ angeordneten Impfkolben /19/ anpassenden rückführenden Feder /33/ in Betätigungsverbindung steht, weiterhin dass der Aufziehgriff /39/ mittels des Aufziehschaftes /42/ und des kraftlagernden Befestigungsbegrenzers /36/ mit der kraftlagernden Feder /40/ in Betätigungsverbindung steht, die während Betrieb des kraftlagernden Befestigungsbegrenzers /36/, weiterhin des Federgehäuses /34/ am verlängerten Schaft der Impfkolbens /19/ angeschlossen ist.

2. Impfgerät nach Anspruch 1, **dadurch gekennzeichnet,** dass ein mit der die regelbare Einschusstiefe des Impfstoffes bestimmenden kraftlagernden Feder /40/ in Verbindung stehender Einschusstiefenregler /44/ mit Kalibriereinteilung vorgesehen ist.

3. Impfgerät nach Anspruch 1 oder 2, **dadurch gekennzeichnet,** dass am Gerätekörper /32/ ein Mischungeinstellkalibrierer /17/ angeordnet ist, dessen Mischungeinstellbefestigungsschraube /16/ an einem Mischungeinstellbefestigungsbolzen /15/ angepasst ist.

4. Impfgerät nach einem der Ansprüche 1 bis 3, **dadurch gekennzeichnet,** dass der am Gerätekörper /32/ angeordnete Aufziehgriff /39/ einen vom Aufziehschaft /42/ grösseren Durchmesser hat.

5. Impfgerät nach einem der Ansprüche 1 bis 4, **dadurch gekennzeichnet,** dass der Anfangsdurchschnitt des Impfkopf-Impfstoffendbeschleunigers /8/ 3 bis 7 mm, zweckmässig 6 mm beträgt und sein enger Querschnitt 0,1 bis 0,2 mm, zweckmässig 0,12 mm ist.

Fig.2

Fig.1

0 286 798